# EUROPEAN PATENT APPLICATION

(11) **EP 4 365 302 A1**
(43) Date of publication of application: **08.05.2024**
(21) Application number: 21951958.4
(22) Date of filing: 23.12.2021
(51) Int. Cl.: C12Q 1/02, C12N 5/071

(54) **DRUG EVALUATION METHOD**

(30) Priority: 30.07.2021 JP 2021125479
(71) Applicant: Ushio Denki Kabushiki Kaisha, Tokyo 100-8150 (JP)
(72) Inventor: YAMANAKA, Makoto, Tokyo 100-8150 (JP)
(74) Representative: Maiwald GmbH
(86) International application number: PCT/JP2021/047955
(87) International publication number: WO 2023/007765

(57) **Abstract**

Disclosed herein is a drug evaluation method capable of evaluating a drug by collecting bile not at a specific point in time but continuously in an environment close to in vivo conditions. The drug evaluation method includes: a step 1 in which hepatocytes and a culture medium are introduced into a microfluidic device; a step 2 in which after the step 1, the hepatocytes are cultured until bile can be excreted therefrom; a step 3 in which after the step 2, a drug is administered to the hepatocytes; and a step 4 in which after the step 3, the bile excreted from the hepatocytes is collected.

## Description

### TECHNICAL FIELD

The present invention relates to a drug evaluation method.

### BACKGROUND ART

In the liver, drugs or other compounds are absorbed into and metabolized by hepatocytes. Hepatic metabolites are excreted into bile canaliculi, reach the gallbladder, and are then discharged from the body.

There are some conventionally-known methods for collecting and analyzing bile secreted from hepatocytes. For example, a method is known in which hepatocytes are cultured on a membrane by sandwich culture method to form tiny bile ducts, and bile secreted from the tiny bile ducts is collected (Patent Document 1 mentioned below). Further, for example, a method is known in which bile is collected from a test animal such as a mouse by directly discharging bile from the body through a catheter inserted into the biliary tract.

### PRIOR ART DOCUMENT

### PATENT DOCUMENT

Patent Document 1: JP-A-2013-17411

### SUMMARY OF THE INVENTION

### PROBLEMS TO BE SOLVED BY THE INVENTION

In the case of sandwich culture method, there is a problem that the life-span of hepatocytes is short (e.g., about 10 days). Further, since sandwich culture method is performed in a state of biliary obstruction where bile is not excreted, intercellular connections need to be broken to collect bile, and therefore continuous analysis (analysis in a state where cells are alive) cannot be performed. That is, sandwich culture method cannot simulate continuous excretion of bile from the human liver. Specifically, sandwich culture method can evaluate bile at a specific point in time but cannot evaluate liver damage caused by administration of a drug for a long period of time.

Animal testing has a problem that drug response varies among individuals of each species, and requires an implantation technique of high difficulty level and know-how to handle animals. First of all, since animals are used, it is not known whether a drug shows the same behavior as when administered to humans.

For these reasons, there is a need for preclinical confirmation of drug safety by continuously evaluating drug efficacy, long-term side effects caused by drug administration, and pharmacokinetics in an environment close to in vivo conditions for a predetermined period of time.

In light of the above problems, it is an object of the present invention to provide a drug evaluation method capable of evaluating a drug by collecting bile not at a specific point in time but continuously in an environment close to in vivo conditions.

### MEANS FOR SOLVING THE PROBLEMS

The present invention is directed to a drug evaluation method including:
a step 1 in which hepatocytes and a culture medium are introduced into a microfluidic device;
a step 2 in which after the step 1, the hepatocytes are cultured until bile can be excreted therefrom;
a step 3 in which after the step 2, a drug is administered to the hepatocytes; and
a step 4 in which after the step 3, the bile excreted from the hepatocytes is collected.

Such a configuration makes it possible to collect bile excreted from hepatocytes while continuing to culture the hepatocytes, and therefore bile can be collected not at a specific point in time but continuously in an environment close to in vivo conditions.

The drug evaluation method according to the present invention may include a step 5 in which the bile collected in the step 4 is analyzed and a step 6 in which pharmacokinetics are evaluated from an analytical result obtained in the step 5.

Such a configuration makes it possible to continuously evaluate pharmacokinetics for a long period of time.

In the drug evaluation method according to the present invention, the step 6 may be a step of evaluating at least one selected from a structure of the drug excreted into bile, a component composition of the drug excreted into bile, a rate parameter of biliary excretion, continuous temporal changes in excretion rate and excretion amount, and a continuous temporal change in metabolism/excretion.

In the drug evaluation method according to the present invention, the step 4 may continuously be performed for one day or more.

In the drug evaluation method according to the present invention, the step 5 may be performed at at least two time points temporally independent from each other.

Such configurations make it possible, when hepatocytes are exposed to a drug for a long period of time, to understand damage to the hepatocytes.

### BRIEF DESCRIPTION OF THE DRAWINGS

Fig. 1 is a plan view of a microfluidic device according to an embodiment of the present invention.
Fig. 2A is a sectional view of the microfluidic device taken along a line A-A shown in Fig. 1.
Fig. 2B is a sectional view of the microfluidic device taken along a line B-B shown in Fig. 1.
Fig. 2C is a sectional view of the microfluidic device taken along a line C-C shown in Fig. 1.
Fig. 2D is a sectional view of the microfluidic device taken along a line D-D shown in Fig. 1.
Fig. 2E is a sectional view of the microfluidic device taken along a line E-E shown in Fig. 1.
Fig. 3 is a perspective view of a third port and its vicinity.
Fig. 4A is a perspective view of a first substrate.
Fig. 4B is a perspective view of a second substrate.
Fig. 5 is a schematic view for illustrating a drug evaluation method using the microfluidic device.
Fig. 6 is an overall flow diagram of the drug evaluation method.
Fig. 7 is a flow diagram of the drug evaluation method.
Fig. 8 is a flow diagram of a pharmacokinetic (AMDE) study.
Fig 9 is a flow diagram of a toxicity/safety test of a drug.
Fig. 10 is a sectional view for illustrating another usage example of the microfluidic device.

### MODE FOR CARRYING OUT THE INVENTION

A drug evaluation method according to the present invention will be described with reference to the drawings. It should be noted that the drawings disclosed herein merely show schematic illustrations. Namely, the dimensional ratios on the drawings do not necessarily reflect the actual dimensional ratios, and the dimensional ratios are not necessarily the same between the drawings.

### [Structure of microfluidic device]

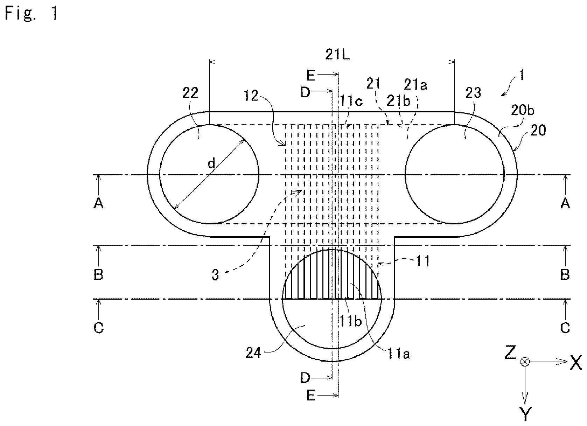

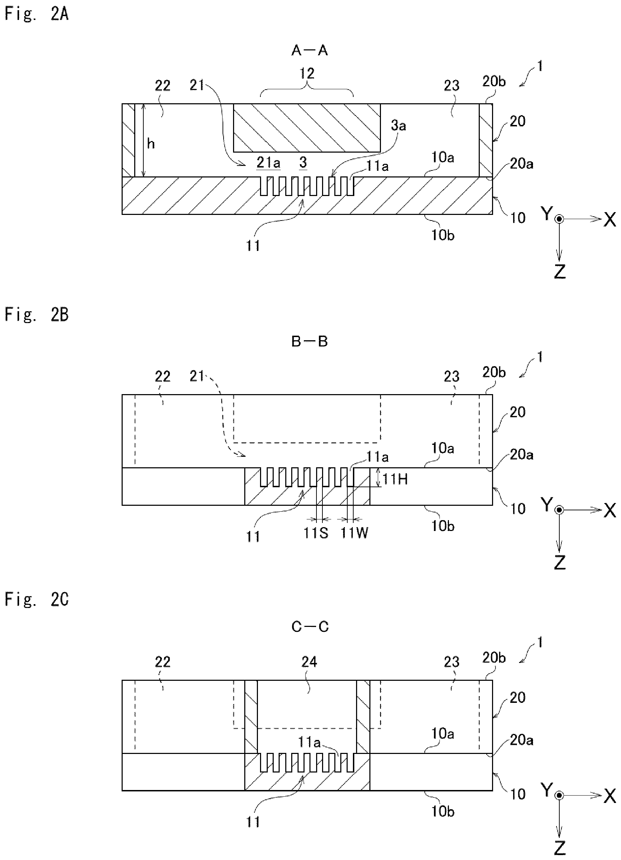

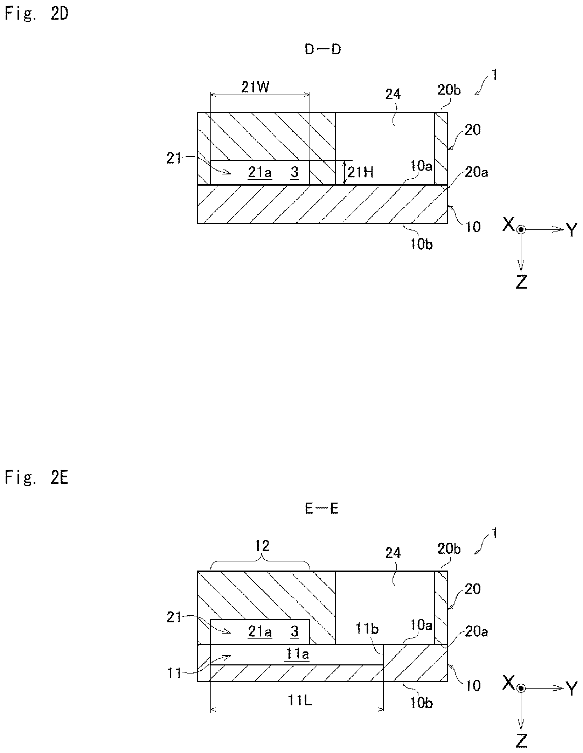

Fig. 1 is a plan view showing an example of a microfluidic device 1 for use in the drug evaluation method. Fig. 2A is a sectional view of the microfluidic device 1 taken along a line A-A shown in Fig. 1. Fig. 2B is a sectional view of the microfluidic device 1 taken along a line B-B shown in Fig. 1. Fig. 2C is a sectional view of the microfluidic device 1 taken along a line C-C shown in Fig. 1. Fig. 2D is a sectional view of the microfluidic device 1 taken along a line D-D shown in Fig. 1. Fig. 2E is a sectional view of the microfluidic device 1 taken along a line E-E shown in Fig. 1.

The microfluidic device 1 is formed by stacking a second substrate 20 on a first substrate 10 in such a manner that one principal surface 20a of the second substrate 20 partially comes into contact with one principal surface 10a of the first substrate 10 and bonding them together. The principal surface refers to one of surfaces constituting the substrate 10 or 20 and having a much larger area than other surfaces. The substrate 10 or 20 has two principal surfaces, and these two principal surfaces are opposed to each other.

The principal surface 10a of the first substrate 10 has a plurality of grooves 11a (which will be described later in detail). Further, the principal surface 20a of the second substrate 20 has a recess 21a (which will be described later in detail). The other principal surface 20b of the second substrate 20 is located on the opposite side from the first substrate 10 and has first to third ports 22 to 24 (which will be described later).

In the following description, an XYZ coordinate system is referenced as appropriate in which, in a state where the first substrate 10 and the second substrate 20 are bonded together, a plane parallel to the principal surfaces 10a and 10b of the first substrate 10 and the principal surfaces 20a and 20b of the second substrate 20 is defined as an XY plane and a direction orthogonal to the XY plane is defined as a Z direction.

When it is necessary to make a distinction between positive and negative to express a direction herein, the direction is described with a positive or negative sign, such as "+X direction" or "-X direction". When it is not necessary to make a distinction between positive and negative to express a direction, the direction is simply described as "X direction". Namely, in a case where a direction is simply described as "X direction" herein, both "+X direction" and "-X direction" are included. The same applies to a Y direction and a Z direction. It should be noted that the microfluidic device 1 is usually used in such a manner that the Z direction corresponds to a vertical direction, and the -Z direction corresponds to an upward direction.

Each of the first substrate 10 and the second substrate 20 has principal surfaces that are the same in shape. In the microfluidic device 1 of the present embodiment, the first substrate 10 and the second substrate 20 have an almost T-shape when viewed from the Z direction. The thickness of the second substrate 20 is larger than that of the first substrate 10. The thickness of the first substrate 10 is, for example, 0.1 to 1 mm, and the thickness of the second substrate 20 is, for example, 3 to 10 mm.

The principal surface 10a of the first substrate 10 includes the grooves 11a. The grooves 11a are formed in the principal surface 10a so as to extend in the Y direction. The grooves 11a are formed to be arranged side by side in the X direction. When the first substrate 10 and the second substrate 20 are bonded together, the grooves 11a function as a secondary flow path 11 sandwiched between both of the substrates 10 and 20. The number of the grooves 11a is, for example, 10 to 1000. In Figs. 1 to 3, only 8 grooves 11a are shown for the purpose of illustration.

Each of the grooves 11a has a slit shape that extends in the Y direction so as to have constant width and depth. Each of the grooves 11a has a rectangular sectional shape. However, the sectional shape of each of the grooves 11a is not limited to a rectangular shape, and may be a trapezoidal or semi-elliptical shape such that the width decreases toward the groove bottom.

Each of the grooves 11a has a width 11W (see Fig. 2B) of, for example, 5 to 100 µm in the X direction. In the present embodiment, the width 11W is 50 µm. The width 11W of each of the grooves 11a is a width at an opening that is open to the principal surface 10a. A space 11S (see Fig. 2B) between the adjacent grooves 11a is, for example, one to two times the width 11W. In the present embodiment, the space 11S is 50 µm. Each of the grooves 11a has a depth 11H (see Fig. 2B) of, for example, 50 to 150 µm. In the present embodiment, the depth 11H is 50 µm. Each of the grooves 11a has a length 11L (see Fig. 2E) of, for example, 4 to 20 mm. In the present embodiment, the length 11L is 6 mm.

The principal surface 20a of the second substrate 20 includes the recess 21a. The recess 21a is formed in the principal surface 20a so as to extend in the X direction. When the first substrate 10 and the second substrate 20 are bonded together, the recess 21a functions as a hollow main flow path 21 sandwiched between both of the substrates 10 and 20.

The recess 21a has a slit shape that extends in the X direction so as to have constant width and depth. The recess 21a has a rectangular sectional shape.

The recess 21a has a width 21W (see Fig. 2D) of, for example, 1 to 10 mm in the Y direction. The recess 21a has a depth 21H (see Fig. 2D) of, for example, 0.1 to 5 mm. The recess 21a has a length 21L (see Fig. 1) of, for example, 4 to 40 mm.

By forming the grooves 11a in the principal surface 10a of the first substrate 10 and forming the recess 21a in the principal surface 20a of the second substrate 20, the main flow path 21 is located in a first XY plane and the secondary flow path 11 is located in a second XY plane different from the first XY plane when the first substrate 10 and the second substrate 20 are bonded together. That is, the secondary flow path 11 is adjacent to the main flow path 21 in the Z direction.

The first port 22 is connected to the -X direction-side end of the recess 21a, and is formed to extend from the principal surface 20a toward the principal surface 20b of the second substrate 20 and to penetrate the second substrate 20. The second port 23 is connected to the +X direction-side end of the recess 21a, and is formed to extend from the principal surface 20a toward the principal surface 20b of the second substrate 20 and to penetrate the second substrate 20.

The third port 24 is formed in a position independent from the recess 21a to extend from the principal surface 20a toward the principal surface 20b of the second substrate 20 and to penetrate the second substrate 20. The third port 24 is formed in a position opposed to one ends 11b of the grooves 11a formed in the first substrate 10 in a state where the first substrate 10 and the second substrate 20 are bonded together. This allows the third port 24 to be connected to the one ends 11b of the grooves 11a when the first substrate 10 and the second substrate 20 are bonded together.

The first port 22, the second port 23, and the third port 24 are all cylindrical hollows extending in the Z direction. In the microfluidic device 1 of the present embodiment, the first port 22, the second port 23, and the third port 24 are all the same in diameter d (see Fig. 1), and the diameter d is, for example, 1 to 10 mm. It should be noted that it is not necessary for the first port 22, the second port 23, and the third port 24 to have the same diameter d. The first port 22, the second port 23, and the third port 24 are all the same in height h (see Fig. 2A), and the height h is, for example, 3 to 10 mm. The distance between the centerline of the main flow path 21 (recess 21a) and the center of the third port 24 in the Y direction is, for example, 3 to 10 mm (see Fig. 2E).

The first port 22 and the second port 23 are connected through the main flow path 21. That is, the main flow path 21 can also be said to have the first port 22 and the second port 23 which are connected to the main flow path 21 and extend toward the principal surface 20b.

The first port 22 and the second port 23 have at least one purpose selected from the purpose of supplying a liquid to the microfluidic device 1 and the purpose of discharging a liquid from the microfluidic device 1. For example, the first port 22 and the second port 23 may be used as a supply port and a discharge port, respectively.

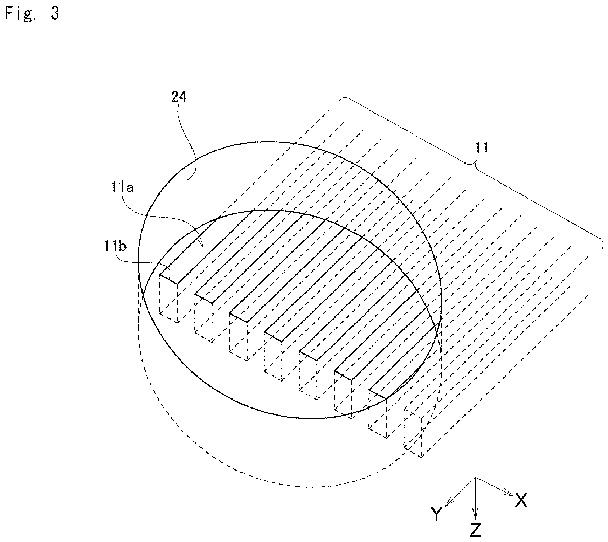

As shown in Fig. 1 and Fig. 3, in a state where the first substrate 10 and the second substrate 20 are bonded together, the grooves 11a are formed in such a manner that the one ends 11b are located on a straight line extending through the center of the third port 24 in the X direction. This allows the one ends 11b of the grooves 11a to communicate with the third port 24. As shown in Fig. 1, other ends 11c of the grooves 11a are located at a -Y direction-side edge 21b of the recess 21a. The grooves 11a have a constant length 11L. Such a constant length 11L of the grooves 11a makes the flow resistance of the grooves 11a constant.

In a state where the first substrate 10 and the second substrate 20 are bonded together, the grooves 11a extend in the Y direction intersecting the recess 21a and partially overlap the recess 21a when viewed from the Z direction. That is, when viewed from the Z direction, the secondary flow path 11 extends in the Y direction intersecting the main flow path 21 and partially overlaps the main flow path 21. Here, an area where the secondary flow path 11 and the main flow path 21 overlap each other when viewed from the Z direction is referred to as an "overlapping area 12".

The grooves 11a are opposed to the recess 21a in a state where the first substrate 10 and the second substrate 20 are bonded together. This allows the grooves 11a to be open to and communicate with the main flow path 21 in the overlapping area 12.

The microfluidic device 1 has a culture space 3 therein, and cells can be cultured in the culture space 3. The culture space 3 is a space within the main flow path 21 in the overlapping area 12. The culture space 3 is opposed to a contact surface 3a (see Fig. 2A) where the secondary flow path 11 is in contact with the main flow path 21 in the overlapping area 12. Although described later in detail, cells introduced into the main flow path 21 adhere to the bottom surface of the culture space 3, specifically the contact surface 3a, more specifically the principal surface 10a of the first substrate 10 in the overlapping area 12. At this time, the cells adhere onto the secondary flow path 11 so as to cover the grooves 11a.

The third port 24 is connected to the secondary flow path 11. That is, the secondary flow path 11 can also be said to have the third port 24 connected to the secondary flow path 11 and extending toward the principal surface 20b.

The third port 24 has the purpose of discharging a liquid in the grooves 11a to the outside. For example, the third port 24 may be used as a collection port to collect a liquid in the grooves 11a.

As described above, the microfluidic device 1 according to the present embodiment has the culture space 3 therein, is capable of culturing cells in the culture space 3, and includes the main flow path 21 and the secondary flow path 11. The main flow path 21 extends in the XY plane. The secondary flow path 11 is adjacent to the main flow path 21 in a direction (Z direction) perpendicular to the XY plane, extends in a direction intersecting the main flow path 21 when viewed from a direction (Z direction) perpendicular to the XY plane, and partially overlaps the main flow path 21. The secondary flow path 11 has the grooves 11a that are open to and communicate with the main flow path 21 in the overlapping area 12 where the secondary flow path 11 overlaps the main flow path 21. The culture space 3 is a space within the main flow path 21, the space being opposed to the contact surface 3a where the secondary flow path 11 is in contact with the main flow path 21 in the overlapping area 12 where the secondary flow path 11 overlaps the main flow path 21.

In the microfluidic device 1 according to the present embodiment, the contact surface 3a preferably has cell adhesiveness. Cell adhesiveness means the property of a surface having a chemical bond as a scaffold for cell adhesion or the property of a surface coatable with an ECM component (such as collagen, gelatin, or laminin), for example, a surface having a functional group such as a hydroxyl group or a carboxy group.

In the microfluidic device 1 according to the present embodiment, it is preferred that the width 21W of the main flow path 21 is larger than a cell to be cultured and the width 11W of each of the grooves 11a is smaller than the cell to be cultured.

### [Method for producing microfluidic device]

Hereinbelow, a method for producing the microfluidic device 1 will be described in detail.

### (Substrate preparing step)

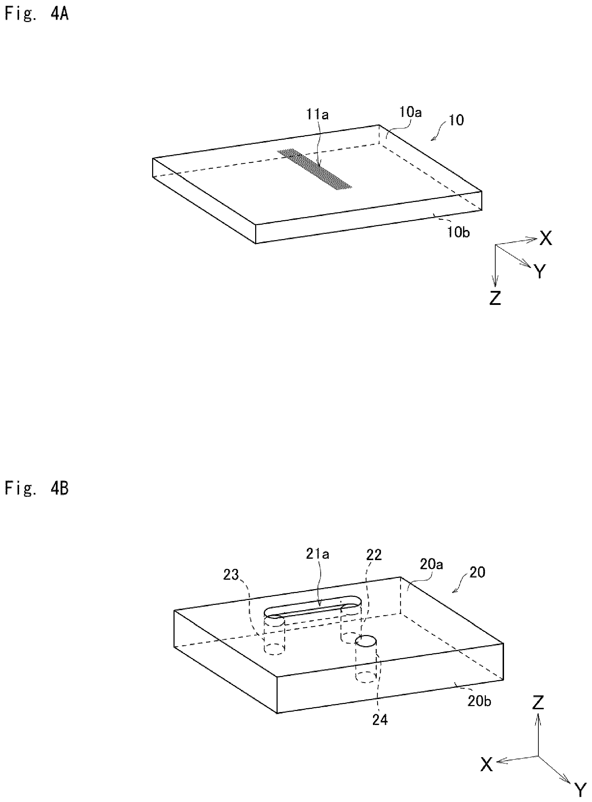

The first substrate 10 and the second substrate 20 to form the microfluidic device 1 are prepared. Fig. 4A is a perspective view of the first substrate 10 before bonding when viewed from the principal surface 10a side, and Fig. 4B is a perspective view of the second substrate 20 before bonding when viewed from the principal surface 20a side.

A material used to form the substrates 10 and 20 is preferably a substantially non-porous material. The "substantially non-porous" herein refers to a state where the apparent surface area of the substrate is approximate to the actual surface area. Examples of a material to form such a non-porous material include an inorganic material such as glass or silicon and a resin material such as polymethyl methacrylate (PMMA), polycarbonate (PC), cycloolefin copolymer (COC), cycloolefin polymer (COP), polystyrene (PS), or silicone. It should be noted that these resin materials may be used in combination of two or more of them. The material used to form the first substrate 10 and the material used to form the second substrate 20 may be different.

The shape of the substrates in the present embodiment will be described. As each of the first substrate 10 and the second substrate 20, a substrate is used which has principal surfaces having the same shape. The thickness of the second substrate 20 is larger than that of the first substrate 10. However, the first substrate 10 and the second substrate 20 may be different in the shape of principal surfaces. For example, the lengthwise dimension and widthwise dimension of principal surface of the first substrate 10 may be larger than those of principal surface of the second substrate 20, or the lengthwise dimension and widthwise dimension of principal surface of the second substrate 20 may be larger than those of principal surface of the first substrate 10. The thickness of the second substrate 20 may be the same as that of the first substrate 10, or the thickness of the second substrate 20 may be smaller than that of the first substrate 10.

The principal surface 10a of the first substrate 10 has the grooves 11a. The grooves 11a are formed by, for example, a combination of a photolithographic process and an etching process, nanoimprinting, laser processing, injection molding, or milling.

The principal surface 20a of the second substrate 20 has the recess 21a. The other principal surface 20b of the second substrate 20 has openings as the first port 22, the second port 23, and the third port 24.

In order to provide openings and recesses in the second substrate 20, for example, a means such as injection molding or cutting work may be used, but an optimum means may be selected depending on the material forming the substrate. For example, as described above, when the second substrate 20 is formed using a resin material such as polymethyl methacrylate (PMMA), polycarbonate (PC), cycloolefin copolymer (COC), cycloolefin polymer (COP), polystyrene (PS), silicone, or acrylic, recesses can easily be formed by injection molding.

### (Substrate bonding step)

The principal surface 10a of the produced first substrate 10 and the principal surface 20a of the produced second substrate 20 are bonded together. A bonding method described below does not require formation of a thin film as an adhesive on the substrate and is performed in the following procedure.

First, the bonding surfaces (10a, 20a) of both of the substrates are subjected to surface activation treatment. As a method of the surface activation treatment, a method including irradiation with ultraviolet rays or a method including contact with plasma gas can be used.

The method including irradiation with ultraviolet rays is performed by, for example, irradiating the principal surface 20a of the second substrate 20 and the principal surface 10a of the first substrate 10 with vacuum ultraviolet rays having a wavelength of 200 nm or less emitted from an ultraviolet light source such as a xenon excimer lamp to emit light having a wavelength of 172 nm. As another example of the ultraviolet light source, a low-pressure mercury lamp having an emission line at 185 nm or a deuterium lamp having an emission line in a wavelength range of 120 to 200 nm can suitably be used. The irradiance of the vacuum ultraviolet rays is, for example, 10 to 500 mW/cm². An irradiation time is appropriately set depending on the type of resin used, and is, for example, 5 to 6 seconds.

The method including contact with plasma gas is performed by generating plasma of a process gas containing nitrogen gas or argon gas as a main component and containing 0.01 to 5 vol% of oxygen gas by atmospheric-pressure plasma and bringing the plasma into contact with the principal surface 20a of the second substrate 20 and the principal surface 10a of the first substrate 10. It is also possible to use a mixed gas of nitrogen gas and clean dry air (CDA). The time of contact with the plasma gas is, for example, 5 to 100 seconds.

Then, a bonding step is performed in which the first substrate 10 and the second substrate 20 are stacked in such a manner that the bonding surfaces (10a, 20a) of both of the substrates subjected to surface activation treatment are in contact with each other, and both of the substrates are bonded together by pressing using a press machine. The bonding step should be performed within a predetermined time, for example, within 10 minutes after the completion of an ultraviolet irradiation step in order to maintain the surface activation state.

If necessary, the bonding step is performed in a thermal environment to achieve tight bonding. In the bonding step, bonding conditions such as heating temperature and pressing force are set depending on the constituent material of the first substrate 10 and the constituent material of the second substrate 20. As for specific conditions, the temperature during pressing is, for example, 40 to 130°C, and the pressing force for bonding is, for example, 0.1 to 10 MPa.

If necessary, a substrate obtained by bonding the first substrate 10 and the second substrate 20 together (hereinafter sometimes referred to as a "bonded substrate") may further be heated for a predetermined time after pressurization for a predetermined time. Even when a portion achieving a satisfactory bonding state and a portion not achieving a satisfactory bonding state are mixed at a bonded interface between the stacked substrates after pressurization, a desired bonding state can be achieved in the portion not achieving a satisfactory bonding state by heating the bonded substrate after pressurization.

After the pressurization of the bonded substrate is maintained for a predetermined time and then stopped, the temperature of the bonded substrate may be increased to and maintained at a predetermined temperature until a desired bonding state is achieved. Here, the predetermined temperature is a temperature at which deformation of the bonded substrate does not occur. For example, a heating temperature is, for example, 40 to 130°C and a heating time is, for example, 60 to 600 seconds.

Then, the microfluidic device 1 in which the second substrate 20 is bonded onto the principal surface 10a of the first substrate 10 is produced through a cooling step.

### [Drug evaluation method]

Hereinbelow, the drug evaluation method using the microfluidic device 1 will be described in detail. However, a microfluidic device used in the drug evaluation method according to the present invention to collect bile is not limited to the above-described microfluidic device 1.

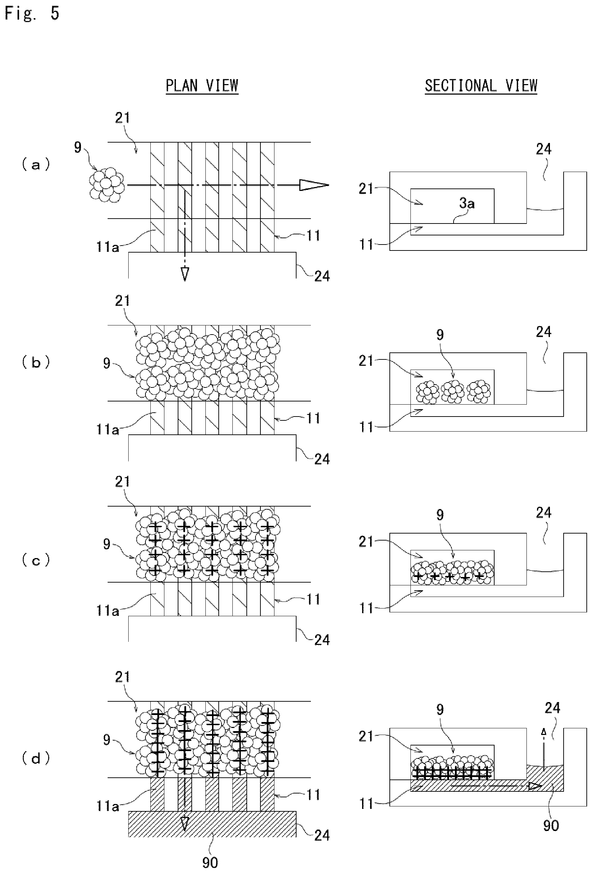

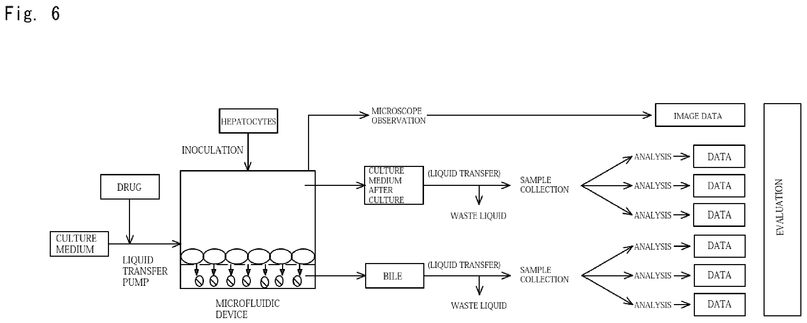

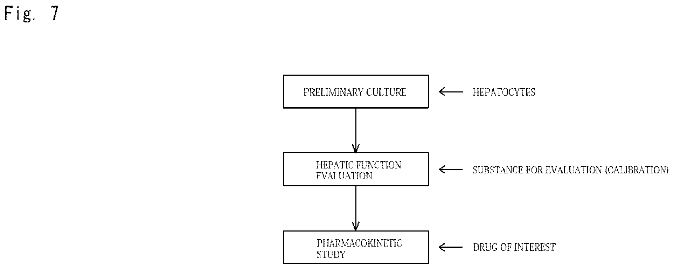

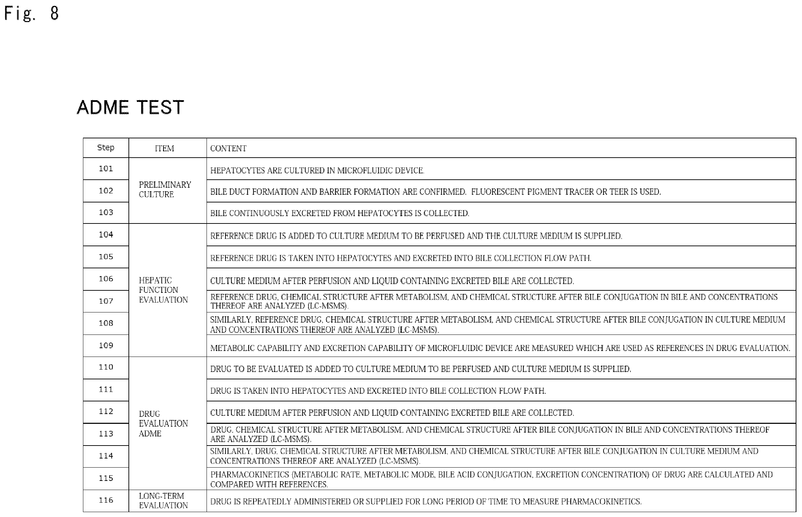

Fig. 5 is a schematic diagram for illustrating the drug evaluation method using the microfluidic device 1, in which plan views are shown on the left side and sectional views are shown on the right side. It should be noted that in the plan views, the grooves 11a are indicated by hatched lines for explanatory convenience. Fig. 6 is an overall flow diagram of the drug evaluation method. Fig. 7 is a flow diagram of the drug evaluation method. Fig. 8 is a flow diagram of a pharmacokinetic (AMDE) study.
(1) First of all, a preliminary culture step shown in Fig. 7 and Fig. 8 is performed. First, as shown in Fig. 5(a) and Fig. 6, hepatocytes 9 and a culture medium (not shown in Fig. 5(a)) are introduced into the microfluidic device 1 (the step 1 in the present invention). Specifically, as shown in Fig. 5(a), the hepatocytes 9 are introduced through the supply port (e.g., the first port 22) to be inoculated in the main flow path 21. At this time, the hepatocytes 9 are introduced as clusters of cells. The size of each of the clusters of cells is, for example, about 50 to 300 µm.
   As shown in Fig. 5(b), the clusters of cells introduced into the main flow path 21 adhere to the bottom surface (contact surface 3a) of the main flow path 21. By setting the width 11W of each of the grooves 11a to be smaller than each of the clusters of cells, the clusters of cells adhere onto the secondary flow path 11.
(2) Then, as shown in Fig. 5(c), the culture medium is injected through the supply port. The culture medium is perfused into the main flow path 21 by collecting it through the discharge port (e.g., the second port 23) to grow the hepatocytes 9. The hepatocytes 9 grow so that most or all of a space above the secondary flow path 11 is filled with them. As the hepatocytes 9 grow, tiny bile ducts (bile canaliculi) are formed in the hepatocytes 9. Until the hepatocytes 9 can excrete bile, culture of the hepatocytes 9 is continued by perfusing the culture medium (the step 2 in the present invention, Step 101 shown in Fig. 8). Then, bile duct formation and barrier formation are confirmed using a fluorescent pigment tracer or TEER (Step 102 shown in Fig. 8). Further, bile continuously excreted from the hepatocytes 9 is collected (Step 103 shown in Fig. 8).

After the step 2, a hepatic function evaluation step shown in Fig. 7 and Fig. 8 is performed. The hepatic function evaluation step is performed to discard the microfluidic device 1 whose hepatic function is below a certain level to perform a drug evaluation described later using the microfluidic device 1 whose haptic function is at or above the certain level.

The hepatic function evaluation step is performed through the following steps. First, a reference drug is added and supplied to a culture medium to be perfused (Step 104 shown in Fig. 8). Then, the reference drug is taken into the hepatocytes 9, and bile is excreted into the bile collection flow path (grooves 11a) (Step 105 shown in Fig. 8). Then, the culture medium after perfusion and a liquid containing the excreted bile are collected (Step 106 shown in Fig. 8). Then, the reference drug, a chemical structure after metabolism, and a chemical structure after bile conjugation in the collected bile and their concentrations are analyzed by, for example, a liquid chromatography mass spectrometer (Step 107 shown in Fig. 8). Similarly, the reference drug, a chemical structure after metabolism, and a chemical structure after bile conjugation in the collected culture medium and their concentrations are analyzed by, for example, a liquid chromatography mass spectrometer (Step 108 shown in Fig. 8). Then, the metabolic capability and excretion capability of the microfluidic device 1 are measured (Step 109 shown in Fig. 8). These are used as references in drug evaluation described later. At this time, as shown in Fig. 6, a cell culture state may also be observed with a microscope as a reference.

(3) Then, a drug evaluation step shown in Fig. 7 and Fig. 8 is performed. First, as shown in Fig. 6, a physiologically active substance and a drug to be evaluated are charged through the supply port into the main flow path 21 in which the hepatocytes 9 that can excrete bile are cultured (the step 3 in the present invention, Step 110 shown in Fig. 8). The drug is taken into the hepatocytes 9, and bile is excreted into the bile collection flow path (grooves 11a) (Step 111 shown in Fig. 8).

(4) Then, as shown in Fig. 6, the culture medium after perfusion and a liquid containing the excreted bile are collected (Step 112 shown in Fig. 8). As shown in Fig. 5(d), bile 90 excreted from the tiny bile ducts flows into the grooves 11a of the secondary flow path 11 and is collected through the collection port (e.g., the third port 24) (the step 4 in the present invention). Since most or all of the space above the secondary flow path 11 is filled with the hepatocytes 9 at this time, it is possible to almost completely or completely prevent the culture medium in the main flow path 21 from flowing into the secondary flow path 11. In the drug evaluation method of the present embodiment, it is not necessary to break intercellular connections when the bile 90 is collected, and therefore the bile 90 can be collected while the hepatocytes 9 are continued to be cultured. Therefore, the bile 90 can be collected not at a specific point in time but continuously in an environment close to in vivo conditions.

The step 4 of collecting the bile 90 is preferably performed continuously for one day (24 hours) or more. The phrase "the step 4 of collecting the bile 90 is performed continuously for one day or more" herein refers not only to continuously collecting the bile 90 for one day or more but also to collecting the bile 90 two or more times for a time period of one day or more, such as a case where after the first collection of the bile 90, the second collection of the bile 90 is performed after a lapse of a predetermined number of days from the first collection. The reason why the bile 90 is continuously collected over a relatively long time span is to understand damage to the hepatocytes 9 due to long-term exposure to the drug.

(5) Then, as shown in Fig. 6, the bile 90 collected through the collection port is analyzed (the step 5 in the present invention). The step 5 is performed by, for example, gas chromatography or liquid chromatography. Specifically, the reference drug, a chemical structure after metabolism, and a chemical structure after bile conjugation in the collected bile and their concentrations are analyzed by, for example, a liquid chromatography mass spectrometer (Step 113 shown in Fig. 8). Similarly, the reference drug, a chemical structure after metabolism, and a chemical structure after bile conjugation in the collected culture medium and their concentrations are analyzed by, for example, a liquid chromatography mass spectrometer (Step 114 shown in Fig. 8).

The step 5 is preferably performed at at least two time points temporally independent from each other. Specifically, the collected bile 90 is preferably analyzed at at least two time points temporally independent from each other. For example, the bile 90 is collected and analyzed every other day or every other week.

(6) Then, as shown in Fig. 6, pharmacokinetics are evaluated from an analytical result obtained in the step 5 (the step 6 in the present invention). Specifically, pharmacokinetics (metabolic rate, metabolic mode, bile acid conjugation, excretion concentration) of the drug are calculated and compared with the references obtained by the above-described hepatic function evaluation to evaluate the pharmacokinetics (Step 115 shown in Fig. 8). Further, long-term evaluation of pharmacokinetics can be performed by repeated administration or long-term supply of the drug (Step 116 shown in Fig. 8).

Meanwhile, in pharmacokinetics, the behavior of a drug in a living body is mainly considered from the time-shift of blood level of the drug. Elements used therefor are called pharmacokinetic parameters. Examples of the pharmacokinetic parameters include maximum drug concentration (Cmax), minimum drug concentration (Cmin), time to maximum concentration (Tmax), blood concentration half-life (T1/2), and area under the blood concentration-time curve (AUC).

In the evaluation of pharmacokinetics of a drug, the structure of the drug excreted into bile, the component composition of the drug excreted into bile, the rate parameter of biliary excretion, continuous temporal changes in excretion rate and excretion amount, and a continuous temporal change in metabolism/excretion are evaluated mainly using liquid chromatography. Hereinbelow, the details thereof will be described.

### a) Structure of drug excreted into bile

The presence of the drug can be confirmed by infrared spectroscopic analysis. Further, the state of chemical structure of a drug compound excreted together with bile is evaluated. Collected bile is analyzed by a GC/MS (gas chromatography mass spectrometer) or a LC/MS/MS (liquid chromatography mass spectrometer) to identify the structure of the drug compound to determine whether the structure has changed and to determine, if the structure has changed, how the structure has changed.

This makes it possible to understand how the chemical structure of the drug compound changes due to biliary excretion. The changes of the chemical structure refer to bile acid conjugation, modification or replacement with a functional group, and cleavage of a bond.

### b) Type, distribution, constitution, contents, composition, and tendency of excreted bile or excreted drug

The change of the chemical structure described in the above a) is not limited to one type. The chemical structure of the drug compound changes at various sites so that various derivative compounds are produced.

Further, bile acid is also a mixture of a plurality kinds of substances. If the change of the composition (the types and concentrations of the substances) of bile acid can be analyzed and linked to the physiological activity of an existing drug, the physiological activity of the unevaluated drug can be predicted by determining the composition of bile acid.

### c) Rate

Understanding of biliary excretion rate of the drug makes it possible to understand how fast the concentration of the drug in blood reduces, and therefore the blood concentration of the drug at a time point after a lapse of a certain period of time from drug administration can accurately be predicted. Further, by combining with the reduction rate of concentration of the drug in a culture medium, the rate of drug accumulation in cells can also be calculated. This makes it possible to predict a feed rate and a time point at which the drug concentration in hepatocytes exceeds a threshold for non-toxicity. This makes it possible to study a safe dose for humans.

### d) Quantity

The balance of the drug to be excreted into bile can be estimated.

### e) Temporal change

On the basis of this, the efficacy, pharmacokinetics, and toxicity of the drug can be evaluated more multidirectionally by a culture test system.

Bile excretion function is not constant over a long period of time. The function of hepatocytes changes due to the effects of the drug (back action of efficacy, dependence, intoxicated state). The change may occur in several hours (acute) or for several days (chronic). Further, a living body is influenced by a biorhythm. In the case of an in-vitro test, bile excretion function changes depending on the culture state of cells (e.g., the degree of maturation of function, the length of time from the start of culture).

Therefore, it is important to evaluate the change at regular certain time intervals. By continuously collecting bile and analyzing the components of the bile, a test to monitor a temporal change can be performed. Further, a model of long-term drug administration can be evaluated.

It should be noted that from the analytical result of the collected bile, the efficacy or toxicity of the drug can also be evaluated. For example, the effect of the drug on a bile excretion rate can be evaluated by monitoring the bile excretion rate. At this time, a drug that reduces the bile excretion rate inhibits bile excretion (cholestasis). Further, since the concentration of the drug in the liver increases, the drug becomes toxic. On the other hand, a drug that increases the bile excretion rate promotes bile excretion, and therefore the concentration of the drug in the liver reduces so that the efficacy of the drug appears.

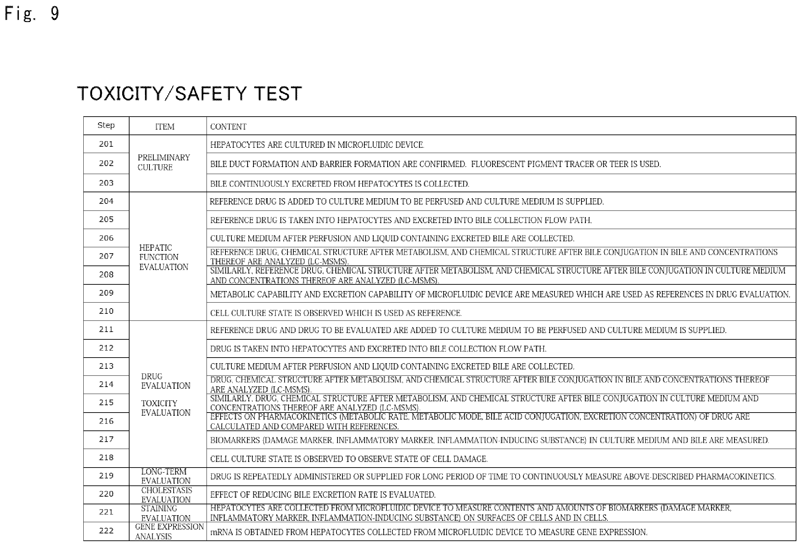

Fig 9 is a flow diagram of a toxicity/safety test of a drug. First, a preliminary culture step is performed. Steps 201 to 203 are the same as Steps 101 to 103 shown in Fig. 8, and therefore the description thereof will not be repeated.

Then, a hepatic function evaluation step is performed. Steps 204 to 209 are the same as Steps 104 to 109 shown in Fig. 8, and therefore the description thereof will not be repeated. In Step 210, a cell culture state is observed with a microscope which is used as a reference.

Then, a drug evaluation step is performed. Steps 211 to 216 are the same as Steps 110 to 115 shown in Fig. 8, and therefore the description thereof will not be repeated. In Step 217, biomarkers (damage marker, inflammatory marker, inflammation-inducing substance) in the culture medium and the bile are measured. In Step 218, a cell culture state is observed to observe the state of cell damage.

In Step 219, long-term evaluation of pharmacokinetics can be performed by repeated administration or long-term supply of the drug.

Further, in Step 220, the bile excretion rate-reducing effect of the drug may be evaluated.

Further, in Step 221, the hepatocytes 9 may be collected from the microfluidic device 1 to measure the contents and amounts of the biomarkers (damage marker, inflammatory marker, inflammation-inducing substance) on the surfaces of the cells and in the cells.

Further, in Step 222, mRNA may be obtained from the hepatocytes 9 collected from the microfluidic device 1 to measure gene expression.

Further, toxicity of the drug can also be evaluated by directly observing the cells as in the case of Step 218. The amount of reduction in the number of bile ducts formed from a normal state, the amount of increase in the number of dead cells, and the proper functioning of paths for bile excretion can quantitatively be evaluated by determining the presence or absence of a fluorescent probe accumulated in the cells.

Although the embodiments according to the present invention have been described above with reference to the drawings, it should be understood that specific configurations are not limited to these embodiments. The scope of the present invention is indicated not only by the above description of the embodiments but also by the claims, and all changes which come within the meaning and range of equivalency of the claims are therefore intended to be embraced therein.

The structure adopted in each of the above embodiments can be adopted in any other embodiment. The specific configuration of each component is not limited only to that in the above-described embodiments, and various modifications are possible without departing from the spirit of the present invention.

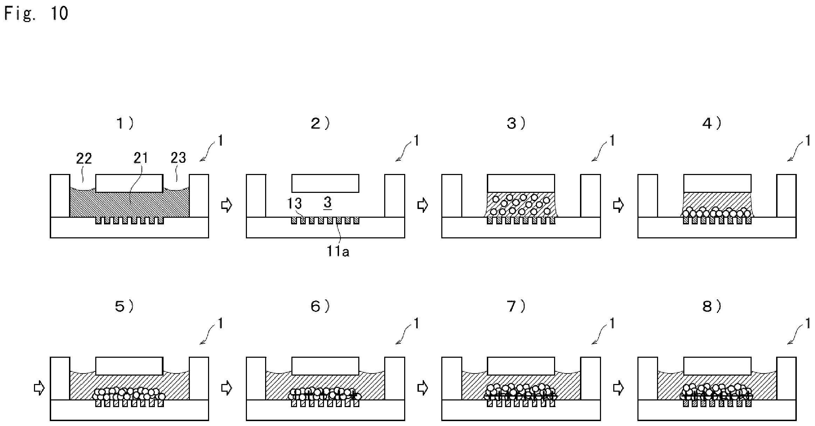

Fig. 10 is a sectional view for illustrating another usage example of the microfluidic device 1. First, 1) the main flow path 21 is filled with a solution for forming a gel. Then, 2) only the solution for forming a gel filled in the grooves 11a is left and gelatinized. A gel 13 may be, for example, a collagen gel. A collagen gel is a gel constituted from collagen fibers. Filling the grooves 11a with the gel 13 makes it possible to culture cells in the culture space 3 even when the cells are smaller than the width 11W of each of the grooves 11a.

Then, 3) hepatocytes are inoculated. Then, 4) the hepatocytes are fixed to the culture space 3 (30 minutes to 1 hour). Specifically, the hepatocytes are allowed to adhere to the contact surface 3a as the bottom surface of the culture space 3.

Then, 5) the first port 22 and the second port 23 are also filled with a culture medium to grow the hepatocytes to obtain hepatic tissue (several days).

Then, 6) bile canaliculi are formed in the hepatic tissue. Then, 7) the bile canaliculi open to the grooves 11a. Then, 8) bile is discharged into the grooves 11a.

It should be noted that the gel 13 may be removed after the cells grow to a size such that they do not fall into the grooves 11a. The gel 13 can be removed by any appropriate method such as spontaneous decomposition, thermal dissolution, or enzymatic decomposition. Even after the gel 13 is removed, membranes or the like of the cells cultured in the culture space 3 are supported by the principal surface 10a between the adjacent grooves 11a, and therefore the form of the cells is maintained.

### DESCRIPTION OF REFERENCE SIGNS

- 1: Microfluidic device
- 3: Culture space
- 3a: Contact surface
- 9: Hepatocyte
- 90: Bile
- 11: Secondary flow path
- 11a: Groove
- 12: Overlapping area
- 21: Main flow path
- 21a: Recess
- 22: First port
- 23: Second port
- 24: Third port

## Claims

1. A drug evaluation method comprising:
a step 1 in which hepatocytes and a culture medium are introduced into a microfluidic device;
a step 2 in which after the step 1, the hepatocytes are cultured until bile can be excreted therefrom;
a step 3 in which after the step 2, a drug is administered to the hepatocytes; and
a step 4 in which after the step 3, the bile excreted from the hepatocytes is collected.

2. The drug evaluation method according to claim 1, wherein in the step 4, bile is collected while the hepatocytes are continued to be cultured.

3. The drug evaluation method according to claim 1, comprising:
a step 5 in which the bile collected in the step 4 is analyzed; and
a step 6 in which pharmacokinetics are evaluated from an analytical result obtained in the step 5.

4. The drug evaluation method according to claim 3, wherein the step 6 is a step of evaluating at least one selected from a structure of the drug excreted into bile, a component composition of the drug excreted into bile, a rate parameter of biliary excretion, continuous temporal changes in excretion rate and excretion amount, and a continuous temporal change in metabolism/excretion.

5. The drug evaluation method according to claim 2, wherein the step 4 is continuously performed for one day or more.

6. The drug evaluation method according to claim 3, wherein the step 5 is performed at at least two time points temporally independent from each other.

7. The drug evaluation method according to claim 3, wherein the step 5 is performed by gas chromatography or liquid chromatography.
